# EUROPEAN PATENT SPECIFICATION

(11) **EP 2 334 561 B1**
(45) Date of publication and mention of the grant of the patent: **09.01.2013**
(21) Application number: 09787057.0
(22) Date of filing: 31.08.2009
(51) Int. Cl.: B65B 31/08, B65B 55/02, B65D 1/36, B65D 81/20

(54) **SYSTEM, METHOD AND DEVICE FOR STERILIZATION AND PACKAGING FOR USE THEREFOR**
SYSTEM, VERFAHREN UND VORRICHTUNG ZUR STERILISIERUNG UND VERPACKUNG DAFÜR
SYSTÈME, PROCÉDÉ ET DISPOSITIF DE STÉRILISATION ET DE CONDITIONNEMENT POUR UTILISATION DE CELUI-CI

(30) Priority: 29.08.2008 BE 200800481
(43) Date of publication of application: 22.06.2011
(73) Proprietor: Ryckewaert, Jan Jozef, 8600 Diksmuide (BE); Wallays, Johan Daniël, 8900 Ieper (BE)
(72) Inventor: Ryckewaert, Jan Jozef, 8600 Diksmuide (BE); Wallays, Johan Daniël, 8900 Ieper (BE)
(74) Representative: Duyver, Jurgen Martha Herman
(86) International application number: PCT/IB2009/053794
(87) International publication number: WO 2010/023640

(56) References cited:
- EP-A- 1 657 158
- WO-A-00/41947
- WO-A-2007/108732

## Description

The present invention relates on one hand to a system for sterilization according to the preamble of the first conclusion. On the other hand this invention relates to a method and device for sterilization and a packaging for sterilization for use with the system, the method and/or the device.

Preserving food is a problem that has been concerning people for centuries. Furthermore, the preservation of food takes an important place in the nutrition of the modern human being. In the past numerous methods were developed to prolong the storage of food. A few known preserving techniques for the preservation of food are amongst others: drying, pickling, sterilizing, smoking, candying, lyophilizing, pasteurizing, etc.

Above mentioned techniques are however insufficient for the preservation of ready-to-serve dishes, with the result that ready-to-serve dishes are distributed until this day with a preservation time that is too limited.

In the search for a method that is able to preserve food longer, especially in the case of ready-to-serve dishes, such as composed meals, the applicant has reached back to sterilization.

In the process of sterilization, a food product is together, possibly with its packaging, subjected to a heath treatment of more than 100°C for a certain time period in which the micro-organisms are killed. For example, canned food products are sterilized at 121° for 20 minutes in a pressure vessel. Higher temperatures and shorter time periods have the same effects, (ex. 3 min. at 134°C). Normally, food products are canned or bottled before sterilization, after which they undergo a heath treatment in a sterilizator with steam or hot water. Sterilizators can operate in a batchwise manner as well as continuously.

The above mentioned conventional method of sterilization however does not allow preservation of a composed meal in its final packaging (usually a packaging made of a synthetic material equipped with a number of compartments to store the different food products) without harming its structure, composition, aroma, taste and presentation. It was determined experimentally that composed meals were often reduced to a sort of pulp after conventional sterilization. Furthermore, after sterilization their aroma and visual presentation were of an inferior quality.

EP 1657158 describes a system including a pressure device cooperating with a connection chamber present in the packaging airtightly closing the packaging.

WO2007108732-A1 for example describes a system for the treatment of a packaging filled with food products in order to prolong their preservation time. Thereto a recipient of a packaging is filled with the food products and closed airtightly and placed in a treatment device. The treatment device comprises a heating element that heats the content of the recipient in order to prolong the preservation time.

This system however does not allow a fine calibration of the treatment conditions of the content of the recipient to the content of the recipient. It is on the other hand only possible to adjust the heath of the content of the recipient.

The inventor realized that in many cases this is insufficient and that next to the temperature, the pressure must also be adjusted and controlled.

It is thus a goal of the present invention to adjust the treatment conditions of the content of the recipient more precisely to the content of the recipient in order to obtain a better sterilization.

Thereto, the present invention is characterized in that the device comprises a pressure device to create an over- and/or an underpressure in the recipient by injecting a fluidum in and/or removing the fluidum out of the recipient through a connecting chamber present in the packaging and closing means to airtightly close the recipient after sterilization by means of the closing element.

By offering the possibility of creating an over- or underpressure in the recipient of the packaging by inserting in and/or removing a fluidum out of the recipient, it is achieved that the pressure in the recipient during sterilization of the content of the recipient can be adjusted more precisely independent of the temperature of the content of the recipient. This allows a more precise calibration of the treatment conditions of the content of the recipient resulting in a better sterilization of the content of the recipient.

By closing the recipient airtightly after sterilization, preferably immediately after sterilization, it is avoided that new micro-organisms will nest in the recipient and the content of the recipient is sterilized under better controlled conditions in the final packaging.

This way, it becomes possible to place the packaging with the recipient closed by the closing element in the device, which is provided to carry the packaging, to sterilize the content of the recipient by heating the content of the recipient by increasing or decreasing the heating element and the pressure in the recipient or at the same time increasing and decreasing the pressure locally in order to create a flow of fluidum in the by the closing element closed recipient (in situ), to close the recipient airtightly after sterilization by the closing element and subsequently take the packaging with sterilized content out of the device and for example, distribute it, storage it, etc.

With content of the recipient in the context of the present invention is for example meant food products or other products or objects such as for example medical instruments, clothing, etc. in their final packaging but also the storage volume of the empty recipient itself. This way it is also possible to treat empty and airtightly closed recipients with this system in order to provide an empty sterile recipient, for example, to take up food products or to be used in a sterile environment such as a laboratory, operating room, etc.

When the recipient more specifically contains food products, especially ready-to-serve meals, it was discovered that it is possible to prolong the preservation of the food products with preservation of aroma, nutritional value, taste and visual presentation.

The above mentioned packaging can be a deep drawn or pressed dish or any other sort of packaging that can be closed by a closing element. The packaging is preferably made of a synthetic material or aluminum and is preferably a disposable packaging for one time use.

The closing element can be for example a foil or a lid that is for example made out of plastic or aluminum.

In preferred embodiments of the system according to the present invention, the pressure device comprises injection means to inject the fluidum through the packaging and/or to remove the fluidum from the connecting chamber which is airtightly closable by the closing element connected to the recipient to create the under- and/or overpressure in the recipient. Such injection means allow injection of the fluidum in and/or removal of the fluidum out of the recipient in a very simple manner. The injection means transport the fluidum preferably through the closing element or the walls of the connecting chamber.

In further preferred embodiments of the system according to the present invention, the connecting chamber is a chamber separated from the recipient. Such a separate chamber allows significantly reducing the risk that the injection means come into direct contact with the content of the recipient.

In further preferred embodiments, of the system according to the present invention, the injection means are provided to couple with the connecting chamber. The inventor has found that such a configuration further simplifies the injection and/or removal of the fluidum. When the coupling happens by pushing material away from the packaging, removing it, piercing it, cutting it, etc. such a configuration allows closing the recipient airtightly even before the sterilization with the system according the present invention so that only during coupling of the injection means to the connecting chamber an opening is created between the injection means and the recipient through the connecting chamber.

In preferred embodiments of the present invention the connecting chamber is provided in the wall of the recipient. Such a configuration offers the advantage that the construction of the closing element becomes simpler.

In a further preferred embodiment of the system according to the present invention the injection means are provided on a movable head, preferably a head that can move in an upward and a downward direction. The closing means furthermore specifically comprise a closing unit slidable over the movable head to close the recipient airtightly, preferably close it temporarily airtightly, more preferably during transport of the fluidum from and to the connecting chamber. This allows that during transport, i.e. injecting and/or removal of the fluidum through the packaging, it is avoided that for example unwanted organisms also move through the packaging and contaminate the content of the recipient.

In further preferred embodiments of the system according to the present invention the above mentioned device further comprises a pushing device for exerting a force on the sides of the packaging opposite the force exerted during the creation of an under- and/or overpressure. The pushing device prevents the packaging, more specifically the connecting chamber and/or the recipient to be damaged during the sterilization process. Such a pushing device also allows packages with a weaker wall, such as a packaging that is made of walls as they are commonly used in the present disposable packages available on the market, to be used in the system according to the present invention.

In further preferred embodiments of the system according to the present invention the pushing device comprises pushing elements to exert a force on the sides of the packaging. Such pushing elements allow in a simple way to compensate for the forces exerted on the walls of the packaging specifically when an overpressure is created in the recipient. Preferably, the pushing elements snuggly surround the recipient so that the walls of the packaging are pressed against the pushing elements when creating an overpressure in the recipient and thus compensate the forces exerted on the walls of the packaging.

In special embodiments of the system according to the present invention the recipient and the connecting chamber are connected with each other through a canal. Preferably the closing units are provided to close the canal by sealing the closing element with the recipient. The above mentioned canal is therefore more preferably provided in the upper edge of the connecting chamber so that when the closing element is pushed on the upper edge by the closing means during welding, the canal is closed.

The invention also relates to a method for sterilization, in which the method comprises the following steps:
- closing the recipient with a closing element;
- creating an over- and/or underpressure in the recipient by injecting a fluidum in or removing the fluidum out of the recipient through a connecting chamber present in the packaging;
- heating the content of the recipient to a predetermined temperature to sterilize the content of the recipient:
   - airtightly closing of the recipient by means of the closing element.

By creating an over- and/or underpressure in the recipient of the packaging by injecting a fluidum in and/or removing the fluidum out of the recipient through the connecting chamber, it is achieved that the pressure in the recipient during sterilization of the content of the recipient can be determined more precisely, where in the past, this was only possible for the temperature of the content of the recipient. This allows a better sterilization to be implemented.

Because after sterilization, preferably immediately after sterilization the recipient is closed airtightly by the closing element, it is avoided that new unwanted organisms nest in the recipient and the content of the recipient is sterilized under better circumstances in their final packaging.

When the method is applied on food products, in particular ready-to-serve meals, it was found that it is possible to preserve the food products longer with preservation of aroma, nutritional value, taste and visual presentation.

In preferred embodiments of the method according to the present method an overpressure is created in the recipient during the heating of the content of the recipient to sterilize the recipient, by injecting the fluidum in the recipient. By creating an overpressure in the recipient of the packaging, a satisfactory sterilization is achieved in a shorter time.

In preferred embodiments of the present invention an underpressure is created in the recipient before the airtightly closing of the recipient through the connecting chamber. This has as a consequence that a longer preservation time can be reached. Furthermore, the method and the system according to the present invention allow the creation of the underpresure after sterilization to be combined with the creation of an overpressure during heating of the content of the recipient in the same device without the necessity of further actions.

In preferred embodiments of the present invention the transport of the fluidum between the connecting chamber, the recipient and a further connecting chamber that is connected airtightly closable by the closing element to the recipient, is achieved by injecting fluidum in the first connecting chamber and removing the fluidum from the second connecting chamber. Such a transport of fluidum through the recipient of the connecting chamber, first connecting chamber, to the next connecting chamber, second connecting chamber, enables a further improvement of the sterilization of the content of the recipient since also places within the recipient which are more difficult to reach, can be reached more easily. It can be noticed that by injecting the fluidum in the first connecting chamber and at the same time removing the fluidum from the second connecting chamber in the recipient, at the same time an overpressure is created at the connection with the first connecting chamber and an underpressure is created at the connection with the second connecting chamber. It is also possible to create a controlled overpressure by squeezing the second connecting chamber.

In preferred embodiments of the present invention a conserving gas is injected in the packaging through the injection means before the closing element is closed. Examples of such conserving gases are for example CO2, nitrogen, etc. Such conserving gases allow the preservation time to be further increased.

In further preferred embodiments the recipient is already airtightly closed by the closing element, for the creation of the over-and/or underpressure in the recipient by injecting a fluidum in or removing the fluidum out of the recipient through the connection chamber, and the recipient is again closed airtightly by means of the closing element after heating of the content of the recipient at a predetermined temperature to sterilize the content of the recipient. Such a method has the advantage that after the recipient is closed airtightly after putting in the food products and before the sterilization, the food products are less in contact with the outer atmosphere during the following steps since the airtight packaging is only temporarily opened during sterilization. In doing so no new micro-organisms can enter the content of the recipient, also before the sterilization.

This invention also relates to a device for sterilization of the content of the recipient of a packaging that is provided to be applied in the system as described here above.

This invention also relates to a packaging with a connecting chamber provided to create an under- and/or overpressure in a by a closing element closed recipient of the packaging, a first connecting chamber connected to the recipient and provided to create an under- and/or overpressure in the recipient by injecting a fluidum in and/or removing the fluidum out of the recipient and a closing element for the airtightly closing of the recipient after an under- and/or overpressure is created in the recipient, characterized in that the packaging is provided with a from the first connecting chamber separated second connecting chamber which is airtightly closable by the closing element connected with the recipient in order to cause a transport of the fluidum between the first connecting chamber, the recipient and the second connecting chamber.

Such a packaging has the advantage that the transport of the fluidum between the connecting chamber, the recipient and the second connecting chamber, allows a better sterilization of the content of the recipient since also places that are difficult to reach in the content of the packaging can be heated and therefore sterilized.

The packaging according to the present invention is preferably provided to be applied in the system described here above.

In preferred embodiments of the packaging according to the present invention the recipient is closed airtightly in order to reduce the risk of unwanted organisms entering the packaging before or after sterilization.

To further clarify the characteristics of this invention and to indicate further advantages and special qualities, a more detailed description of the system and the method for sterilization of food products follows. It is clear that nothing in the following description can be interpreted as a restriction of the protection of this invention as claimed in the claims.

In this description is by means of reference numbers referred to the herewith added drawing whereby:
- figure 1: is an explosion representation of the system according to the invention;
- figures 2 up to and including 9: are a schematic representation of the method according to the invention;
- figure 10 A: is a representation of the inside of a first embodiment of the packaging according to the invention;
- figure 10 B: is a representation of the inside of a second embodiment of the packaging according to the invention;
- figure 11: is a representation of the packaging represented in figure 10 A with closing element (4) and decorative lid (16);
- figure 12: is a detailed representation of the connecting chamber for a packaging with three compartments:
- figure 13: is a top view of the second embodiment of the packaging according to the invention;
- figure 14: is an exploded representation of a preferred embodiment of the system of the invention;
- figure 15: is a top view of the system according to figure 14;
- figure 16 A: is a section of the system according to figure 14 along the axis A-A.
- figure 16 B: a detail of figure 16 A
- figure 17: a bottom view of the exploded representation of figure 14;
- figure 18: is an exploded representation of the system according to figure 14 included in a device;

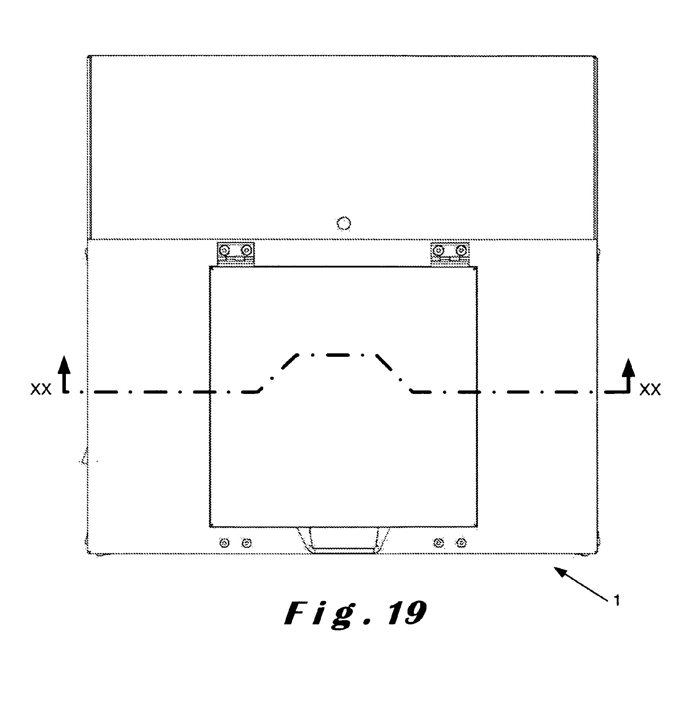

- figure 19: is a top view of the system according to figure 18;
- figure 20: is a section of the system of figure 19 along the axis A-A.

The system according to the invention comprises on one hand a packaging 2 with a recipient 3 closed by a closing element 4 and on the other hand a device 1 for the sterilization of the content of the recipient 3.

The content of the recipient 3 can contain products or objects such as food products, more specifically ready-to-serve meals, medical instruments, more specifically surgical instruments, more specifically a set for a specific medical intervention, such as for example a set of medical instruments, more specifically surgical instruments that are necessary to conduct an appendicectomy, etc... Recipients 3 with such content can subsequently be preserved for a longer time under sterile conditions, which is important for food products to guarantee a long conservation time even if the packaging 2 isn't stored coolly. By providing packages 2 that can sterilely store objects such as medical material, it becomes possible to sterilely store the material sterile for a longer time and for example to transport it over longer distances without increasing the danger to infections.

The recipient 3 can however stay empty as well. In that case the packaging can be used in sterile conditions without contaminating the sterile environment. If for example a sterile recipient is desirable in a sterile environment, such as an operation room or a laboratory, an empty recipient 3 that is sterilized according to the present method, can be used.

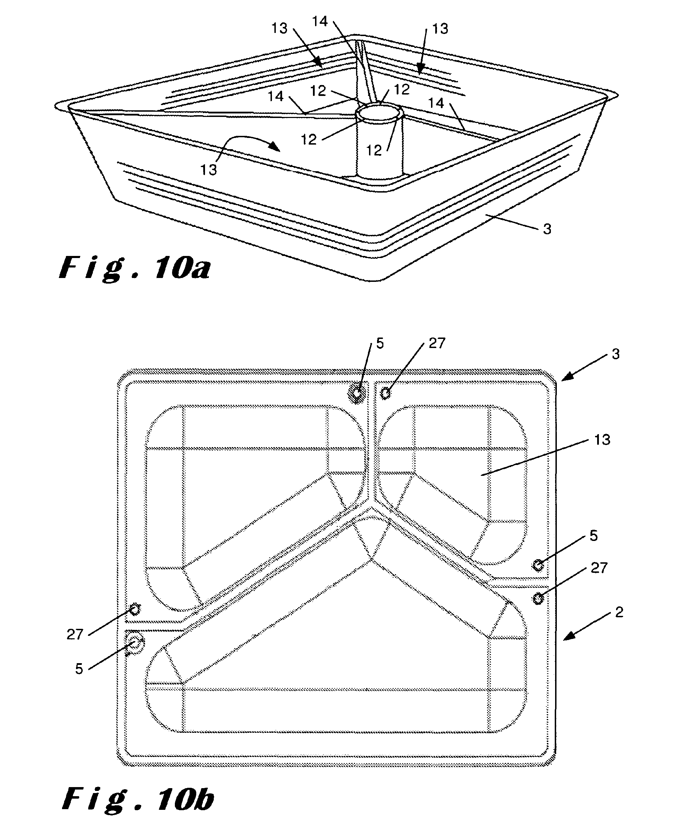

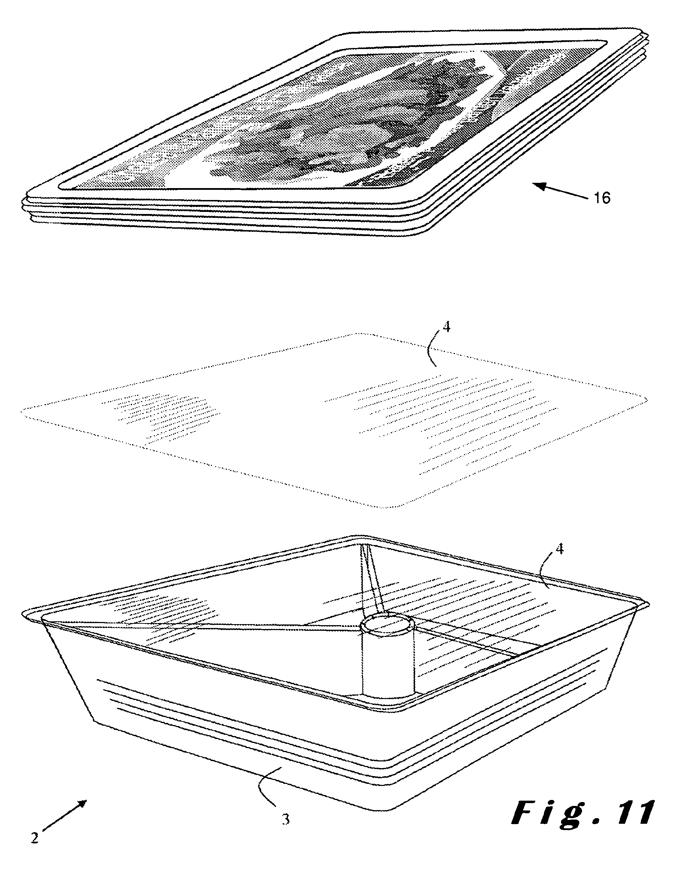

Figures 10 A and 11 show a first embodiment of the packaging 2 according to the invention. The packaging 2 consists of a recipient 3 with a content such as described above. The recipient 3 comprises a bottom and sidewalls extending transversely to the bottom. The recipient is closed by a closing element 4 that also allows a later airtightly closing of the recipient 3. The closing element 4 is preferably a foil that preferably is applied along the entire circumference of the recipient 3, through for example a thermal connection by for example melting (sealing) the recipient and the closing element together along the circumference of the recipient 3. The closing element 4 can however also be a lid that is put on the recipient 3, such as represented in figure 11.

The recipient can comprise one compartment 13, such as represented on figure 10, in which for example different food products or other products or objects can be placed. The different compartments 13 are preferably separated from each other through flanges 14. During the closing of the recipient 3 with the closing element 4, for example by a foil, this foil is preferably also connected with the flanges 14. Preferably, however the different compartments are already separated from each other airtightly before sterilization, for example by connecting the flanges 14 with the closing element 4 before sterilization. By directly separating the different compartments 13, it is achieved that during sterilization, the content of the recipient 3 and thus of the different compartments isn't mixed. This is especially important when the recipient 3 contains food products and has the advantage that taste substances, aroma substances, coloring substances, etc. of the different food products present in the different compartments aren't mixed with each other as a result of which the food products can lose its taste, smell and color. Thus, for example different food products stored in the recipient 3 can be sterilized separately and yet be packed together in a packaging 2 by storing them separately from each other in different compartments of the recipient 3.

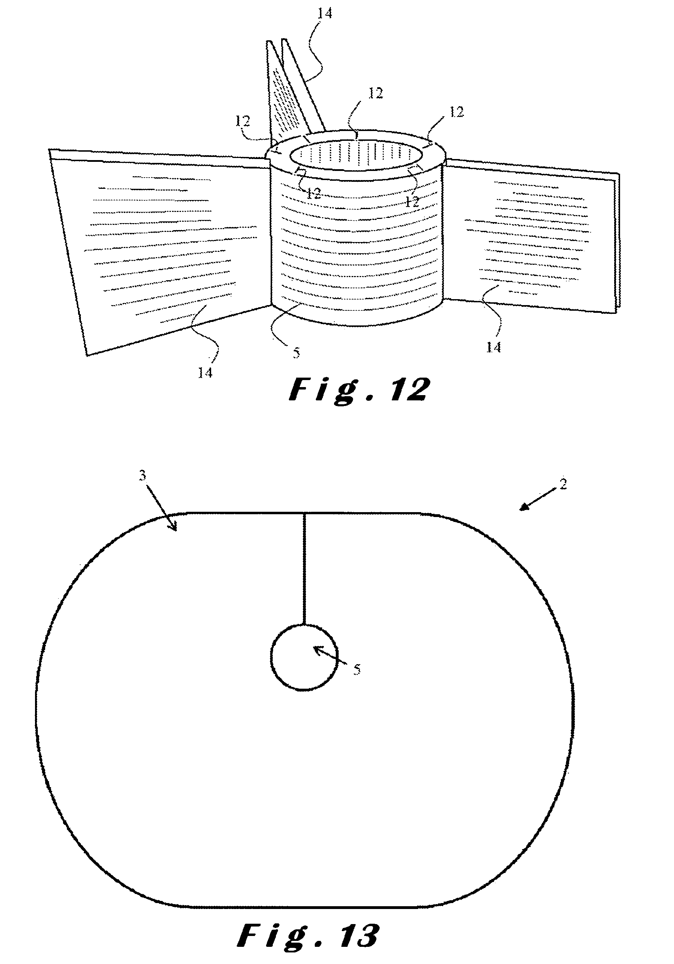

The packaging 2 further comprises, such as specifically shown in figure 12, a connecting chamber 5 that is provided for creating an under- and/or overpressure in the recipient 3 by removing a fluidum out of and/or injecting the fluidum in the recipient 3 through the connecting chamber 5. The connecting chamber 5 is preferably provided in the wall of the recipient 3, preferably extending transversely along the bottom, and has in a first embodiment, more preferably, a height that nearly corresponds with the height of the recipient 3. Preferably the connecting chamber 5 is however a relatively small chamber that extends at the bottom, i.e. opposite of the closing element 4, the recipient 3 in a downward direction, i.e. in a direction away from the closing element 4, and is part of the wall of the recipient 3, as shown in for example figure 10 B. The connecting chamber 5 preferably has a circular cross section but can also be implemented in other forms, such as for example, rectangular, oval, ... The connecting chamber 5 can be provided centrally in the recipient, although this is not central, as shown in figure 10 B. The connecting chamber 5 and the one or more compartments 13 are preferably connected with each other through at least one canal 12. These canals 12 are preferably provided in the upper edge of the connecting chamber 5.

Although the connecting chamber 5 shown in the figures only comprises one single compartment, a connecting chamber 5 can also be provided that comprises multiple compartments. Preferably these compartments of the connecting chamber 5 are each in connection with predetermined compartments of the recipient 3. More preferably the different compartments of the connecting chamber 5 are in connection with specific compartments 13 of the recipient 3. This has as a consequence that the pressure in the different compartments 13 of the recipient 3 can be regulated more individually by setting a different pressure in the different compartments of the connecting chamber 5.

The connecting chamber 5 is preferably a chamber separate from the recipient 3. It is also possible to provide one or more connecting chambers 5, 27 for every compartment 13, such as for example showed in figure 10 B in which every compartment 13 is provided with respectively a first 5 and a second connecting chamber 27.

The second connecting chamber shown in figure 10 B is preferably airtightly closable connected by the closing element 4 to the recipient 3 in order to enable a transport of fluidum between the first connecting chamber 5, the recipient 3 and the second connecting chamber. The second connecting chamber 27 is further constructed analogously to the first connecting chamber 5 and further characteristics of the first connecting chamber 5 can also be applied on the second connecting chamber, for example relating to installation, shape, etc.

Such a second connecting chamber 27 can for example be provided in the shape of an additional chamber, next to the already provided connecting chamber 5, adjacent to the recipient 3 and connected to the recipient 3 by means, of for example, a canal 12. If multiple compartments 13 are provided in the recipient 3, the second connecting chamber 27 can be adjacent to each of the compartments 13. If it is provided that the compartments are organized around a central axis, wherein preferably the connecting chamber 5 is provided, the second connecting chamber 27, in this embodiment preferably, also is provided around the central axis, more preferably around the different compartments 13. Although the second connecting chamber 27 can also consist of a single compartment the second connecting chamber 27 can also comprise multiple compartments, for example a compartment per compartment 13 of the recipient 3. Such a configuration allows the flow of the fluidum through the compartments 13 of the recipient 3 to be controlled individually. It is however also possible to provide multiple second connecting chambers 27, as shown in figure 10 B. Thus it is for example possible, as shown in figure 10 B, to provide respectively a recipient 3 with multiple compartments 13 in which every compartment is connected with a connecting chamber 5 and second connecting chamber 27 as a result of which there are an equal amount of connecting chambers 5 and second connecting chambers 27 as compartments 13 of the recipient 3.

The content of the recipient 3 is sterilized by means of the system according to the invention, more specifically in the device 1 according to this invention.

The device 1 for sterilization of the content of the recipient 3 of the packaging 2 according to this invention comprises a heating element 8 to sterilize the content of the recipient 3 by heating, a pressure device 7 to create an under- or overpressure in the recipient 3 by injecting the fluidum in or removing the fluidum out of the recipient 3, through the connecting chamber 5 of the packaging 2 and closing means 9 to close the recipient 3 airtightly with the closing element 4 after sterilization. Preferably the pressure device 7 comprises one or more injection means 6 to remove the fluidum out and/or inject the fluidum in the connecting chambers 5 through the closing element 4. The connecting chamber 5 is thereby airtightly closed by the closing element 4 connected to the recipient 3 to create the under- and/or overpressure in the recipient 3. The injection means 6 are preferably provided on a movable head 11.

To create an overpressure, a fluidum, such as for example air or any other gas, is injected in the packaging. Hereby the injected fluidum flows from the connecting chamber 5 to the recipient 3, for example through the canals 12 to the different compartments 13 of the recipient 3. To create an underpressure in the recipient 3 the present fluidum will be removed out of the recipient 3 through means of the pressure device 7. The connecting chamber 5, separate of the recipient 3, makes sure the contained food products are not damaged during injection of removal of air or other gases out of the packaging 2.

If the packaging 2 is provided with a second connecting chamber 27, as described here above, the pressure device 7 is preferably also provided to, analogous to the connecting chamber 5, remove the fluidum out or inject the fluidum in the recipient 3 through the second connecting chamber 27 of the packaging 2 and the closing means to close the recipient 3 airtightly after sterilization by means of the closing element. Preferably the pressure device 7 comprises one or multiple injection means 6 to remove the fluidum out or inject the fluidum in the second connecting chamber 27 through the closing element 4. The second connecting chamber 27 is thereby closed airtightly closable by the closing element 4 connected to the recipient 3 to create the under- or overpressure in the recipient 3.

Preferably the pressure device 7 is adapted in such a way that the pressure device 7 is provided to inject fluidum in the connecting chamber 5 and remove the fluidum in the second connecting chamber 27 at the same time in order to cause a transport of the fluidum between the connecting chamber 5, the recipient 3 and the second connecting chamber 27. As discussed previously, such a transport of fluidum has an improved sterilization as a result.

In order to obtain access to the connecting chamber 5, 27 the closing element 4 can thereto be provided of several openings to the connecting chamber 5, 27. By creating an over- or underpressure above these openings by means of the pressure device 7, the fluidum can in this way be injected in or removed out of the connecting chamber 5, 27 and thus in or out of the recipient 3. These openings can however also be provided in the wall of the connecting chamber 5, 27 that is provided in the wall of the recipient 3 and can, more preferably, be provided by cutting away a part of the packaging 2, whereby the packaging 2 is provided of means that allow this, such as cuttable small projections provided on the openings with preferably a weakness in the wall of the packaging 2 on the cutting position in order to simplify the cutting of the small projection. The device 1 and preferably the injection means 6 can also be provided with cutting means that allow the cutting. Preferably the injection means 6 are provided to couple with the connecting chamber 5. For this purpose the injection means 6 and the connecting chamber 5 are for example provided with cooperative screw thread that allows screwing of the injection means 6 onto the connecting chamber 5. Such a screw thread onto the connecting chamber 5 also allows closing the opening to and from the connecting chamber with a cap that is screwable onto the connecting chamber 5.

The injection means 6 can also be provided to pierce the packaging 2, preferably the closing element 4 or the connecting chamber 5, more preferably the closing element 4 near the connecting chamber 5, 27. To create an over- or underpressure in the recipient 3, the injection means 6 are pierced through the packaging 2 near the connecting chamber 5, 27. Subsequently the fluidum is injected or removed through the injection means. Such a shape of the injection means 6 allow the recipient 3 to already be closed airtightly temporarily by the closing element 4 before the packaging 2 is placed in the device 1 and thus before sterilization. Also the piercing of the packaging is simple to execute. This allows the content of the recipient 3 to be further protected, also before sterilization, which is important when the recipient contains for example food products.

To facilitate the piercing of the packaging 2, preferably the connecting chamber 5, 27, the thickness of the wall of the connecting chamber 5, 27 where the injection means will be pierced through the packaging 2, is preferably thinner than the surroundings of that location. Thus, the piercing, cutting, pushing away, etc. of the wall of the connecting chamber 5, 27 is facilitated. The piercing of the connecting chamber 5, 27 can be performed by a piercing movement of the injection means 6 through the wall of the connecting chamber but can also be performed by a cutting movement of the injection means, a hitting movement, etc. The injection means can for example comprise a hitting, cutting, piercing, etc. element combined with a second element that is provided to be stuck in the connecting chamber 5, 27 to inject or remove the fluidum after the hitting, piercing, cutting.

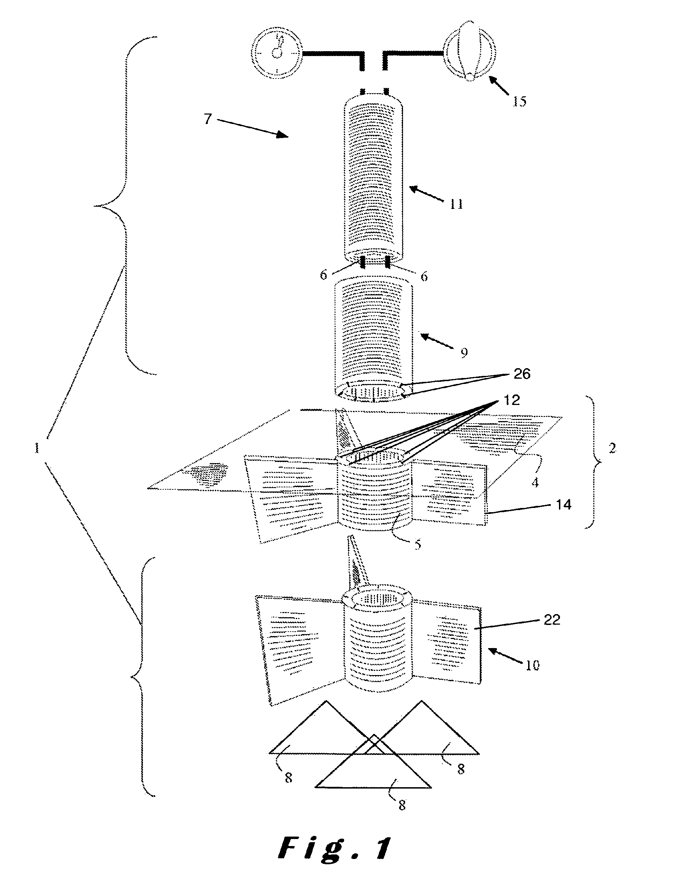

In the system shown in figure 1 the packaging 2 for example has a connecting chamber 5 with a single compartment. The connecting chamber 5 is connected with the different surrounding compartments 13 of the packaging 2.

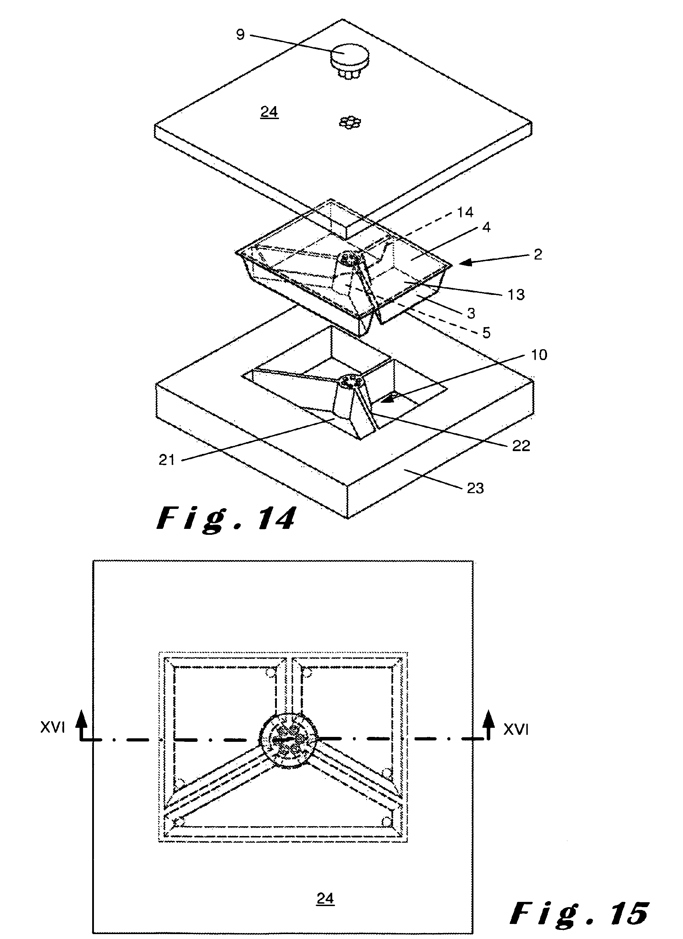

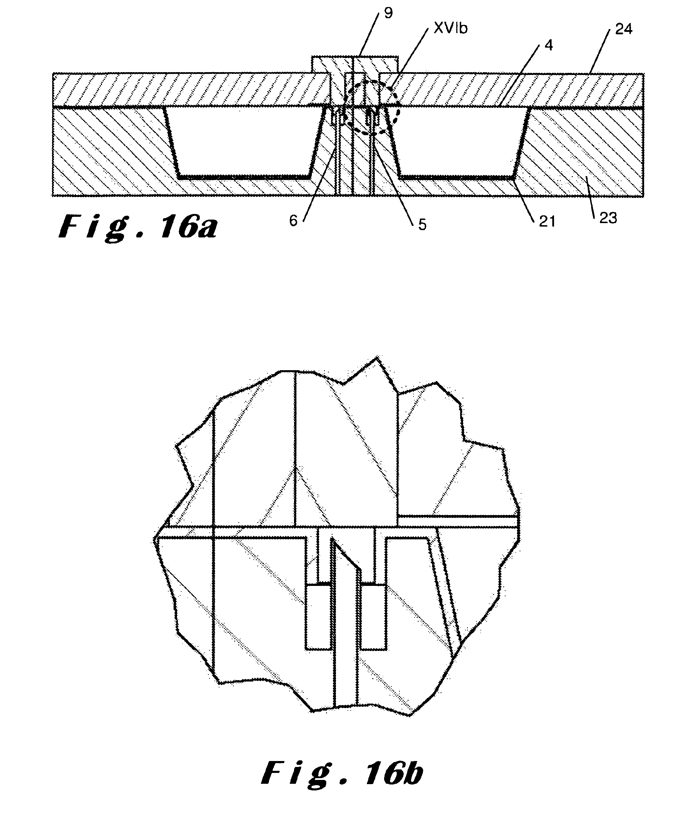

The packaging 2 shown in figures 14-17 has multiple connecting chambers 5, more specifically 6 compartments. The compartments are provided at the bottom of the packaging 2, this means at the side of the packaging 2 positioned opposite of the closing element 4. In accordance with the compartments of the connecting chamber 5, the device 1 comprises several injection means 6, more specifically needles, more specifically six needles that are capable of piercing the compartments of the connecting chamber 5. This is shown in figure 16 B in which a detail is shown of an injection mean 6 that pierces the packaging 2, more specifically a compartment of the connecting chamber 5. Although a single connecting chamber 5 is sufficient in the system and the method according to the present method and for use in the device according to the present invention, it is possible that multiple connecting chambers 5 are also provided in the packaging 2, such as the second connecting chamber 27 in the packaging according to the present invention. These connecting chambers 5 can be adjacent but can also lay spread across the packaging 2.

Between the connecting chambers 5, 27 and the recipient a canal can be provided that is closed if no fluidum is supplied and can be blown open if fluidum is injected in the recipient 3. After sterilization the canal 12 is closed by exerting pressure on the canal 12 and securing the closing element 4 in this position onto the recipient 3, for example by sealing it. It is however also possible that the canals 12 are provided in the shape of recesses that are provided in the upper edge of the connecting chamber 5, 27 that form an opening between the upper edge of the connecting chamber 5, 27 and the closing element 4. In such a case it is for example possible the close the canal 12 by pushing the foil in the canal 12 and subsequently sealing it with the walls of the canal 12.

It can be noticed that before use, the recipient 3 can be completely closed airtightly by the closing element 4 along the contour of the recipient 3. Although during sterilizing the recipient 3 is no longer closed airtightly since the compartments of the connecting chamber 5, 27 are pierced, an airtight closing of the recipient 3 is again possible by closing the recipient 3 airtightly off the connecting chamber 5, 27, in this case by closing the canal 12. If the connecting chamber 5, 27 is located centrally, as shown in figure 15 and figure 1, the airtight closing of the connecting chamber 5, 27 of the surrounding recipient 3 can for example happen by sealing the upright edge around the connecting chamber 5, 27 to the closing element 4. Therefore the canals 12 are airtightly closable by sealing the closing element to the recipient 3.

The injection means 6 are preferably provided an a movable head 11 to move the injection means 6 to and away from the packaging 2.

If the recipient 3 comprises multiple compartments 13, a separate heating part of the heating element 8 can be provided per compartment 13 as for example shown in figure 1. As such the food products placed in the different compartments 13 can be brought to a desired temperature during a desired period . The heating parts of the heating element 8 are for example electrical heating parts, as shown in figure 1. It is however obvious that the heating parts can be adapted to bring the food products placed in the different compartments 13 at temperature through radiation of another known or until now unknown method. By heating the food products during a certain time the micro-organisms in the food products will be killed. Furthermore, enzymes will be neutralised by the heath.

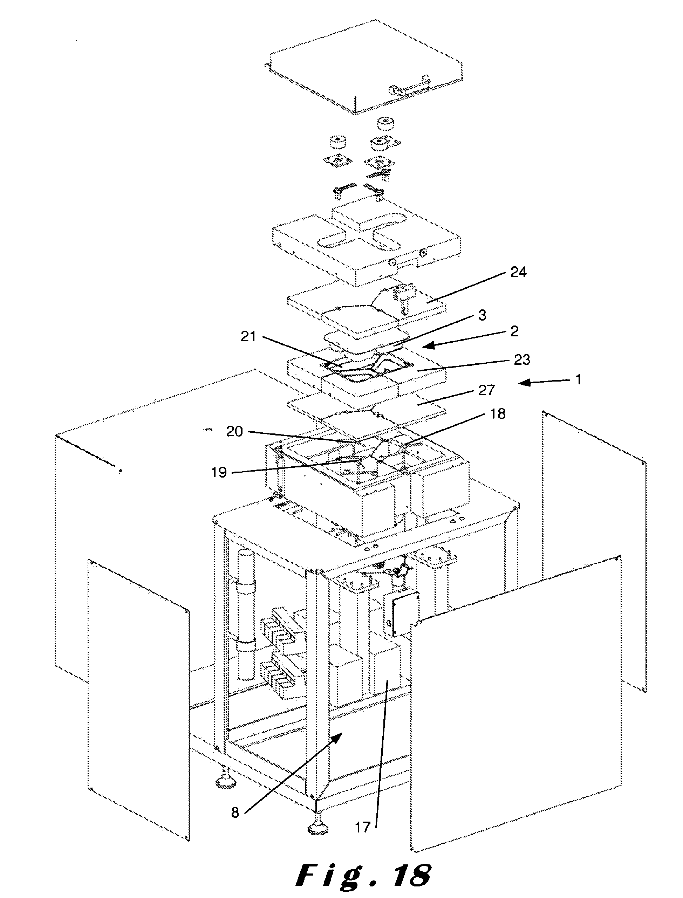

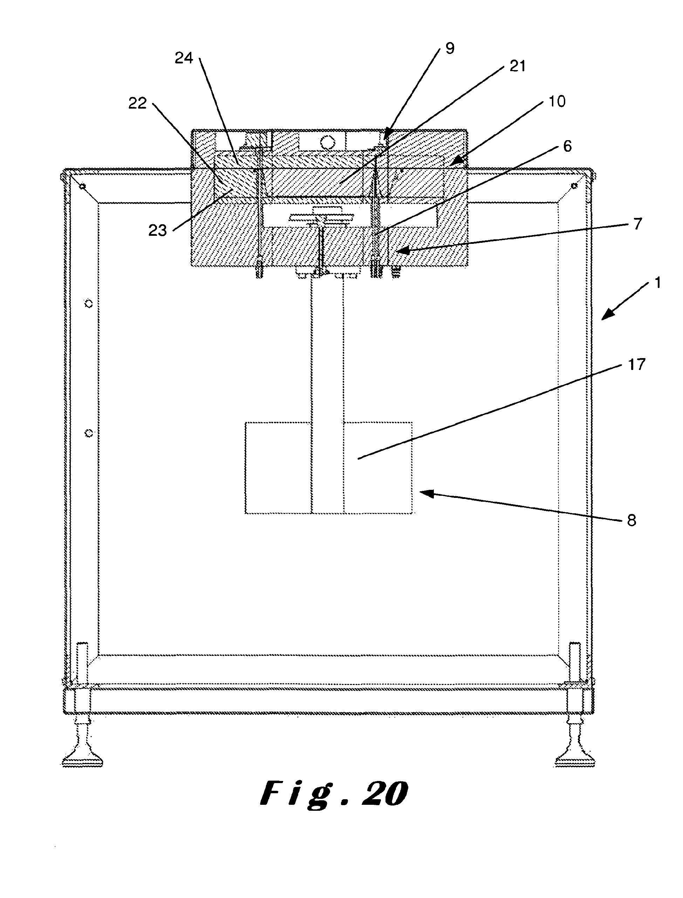

Preferably the heating element 8 comprises a microwave radiation-source 17 as shown in figure 20, preferably a linear continuously variable microwave generator. More preferably a separate heating part is provided per compartment, with further preference a separate microwave source 17, as shown in figure 18. The microwave source 17 preferably comprises a so-called mode-stirrer 18, as shown in figure 20, that allows the microwave radiation emitted by the microwave source to be spread better, and thus to homogenize the distribution of the microwave radiation. More preferably each compartment comprises a separate mode-stirrer 18, 19, 20 so that the homogeneity of the emitted micro-wave radiation further increases in each compartment separately.

The closing means preferably consist of a closing unit 9 slidable over the movable head 1. The closing unit 9 is preferably provided to close the closing element 4 airtightly near the canals 12 at the upper edge of the connecting chamber 5, as discussed above and can therefore for example be provided with a pushing edge for pushing the closing element against the upper edge of the connecting chamber 5 with a shape that snuggly fits the shape of the upper edge of the connecting chamber 5, with thickenings 26 on the pushing edge that cooperate with the shape of the canals 12 in the edge of the connecting chamber 5, as shown in figure 1. Further the closing means are provided to attach the closing element 4 to the recipient 3 and thereto comprise for example a heating element provided to seal the closing element 4, in that case preferably a thereto provided foil, to the upper edges of the recipient 3.

To prevent the packaging 2 from being damaged by for example piercing the closing element 4 or the creating of an over- or underpessure in the packaging 2, the device 1 further comprises a pushing device for exerting a force on the walls of the packaging 2 opposite of the force exerted on the walls of the packaging during creating of the under- or overpressure.

The pushing device 10 preferably comprises pushing elements 22 to exert the force on the walls of the packaging 2. More preferably the pushing elements 22 are formed in such a way that they snuggly fittingly surround the packaging 2. Such an embodiment is for example shown in more detail in figure 10 and comprises preferably a bottom part 23 that supports the recipient 3 and the connecting chamber 5 of the packaging 2, and an upper part 24 that covers the closing element 4. Both parts 13, 24 are preferably made out of PEEK.

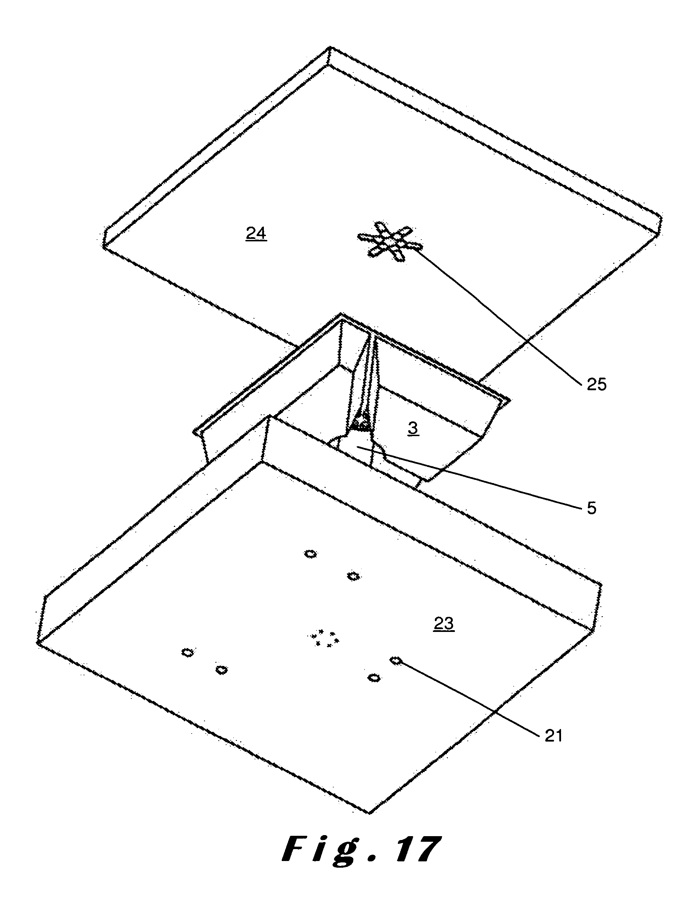

The bottom part 23 shown in figure 17 is provided of openings that allow access to the injection means 6 through the bottom part 23 to the packaging 2. This is however not necessary for the invention and the injection means can for example also be provided above the bottom part 23.

The upper part 24 preferably comprises several recesses that allow the closing element to be locally elevated so that the canal 12 between closing chamber and recipient 3 can be opened when the fluidum is injected in the connecting chamber 5 so that the fluidum can flow to the recipient 3. The recesses 25 are as such dimensioned that they one hand allow the fluidum to open the canal yet on the other hand exert a sufficient amount of force on the packaging 2 in order to compensate for the forced caused by the overpressure.

Preferably the pushing device 10 allows that by creating an overpressure in the recipient 3, the closing element 4 is elevated slightly on the position where the closing element will later be airtightly connected with the recipient 3. Such a construction allows making less accessible places, such as the crack between closing element 4 and the recipient 3, accessible for sterilization.

The device 1 further preferably comprises a cooling device 21 to cool the content of the recipient 3 after heating of the content of the recipient 3 as a part of the sterilization process. The cooling device 21 thereto preferably comprises several canals that allow a cooling fluidum to be led alongside the packaging 2, preferably alongside the recipient 3. The canals are placed in such a way that the cooling fluidum, preferably a cooling liquid, such as for example water, more specifically icewater, can absorb the heath of the content of the recipient 3 so that the content of the recipient 3 is cooled. In a preferred embodiment, the canals of the cooling device are taken up in the pushing elements of the pushing device 10 since these pushing elements preferably surround the packaging, preferably the recipient 3 in a snuggly fit fashion and the canals are preferably positioned closely to the packaging, more specifically the recipient, to better absorb the heath of the content of the recipient 3. Preferably, the cooling fluidum comes in direct contact with the packaging 2, more specifically, with the recipient 3. As such, the cooling fluidum can for example be spraid onto the packaging 2. In order to supply the cooling fluidum to the packaging 2 there are, as shown in figure 17, several openings provided in the bottom part 23 that lead to several canals that touch the packaging 2, preferably at the recipient 3, and allow the cooling fluidum to come in direct contact with the packaging 2, preferably the recipient 3.

If the cooling fluidum comes into direct contact with the packaging 2, more specifically the recipient 3, the heating element 8 is separated from the places where the cooling fluidum flows, the circuit of the cooling fluidum, especially if these are electrically driven. Thereto, preferably a plate is installed between the heating element 8 and the circuit of the cooling fluidum. If the heating element 8 comprises a microwave source 17 to heat the content of the content of the recipient 3, the plate is preferably a glass plate 27, more preferably a quartz glass plate, since such a plate lets microwave pass at a maximal level.

If the heating element 8 comprises a microwave source 17, the device 1 preferably is provided to absorb the radiation emitted by the microwave source 17 so that this can not leave the device. Thus, it is prevented that a user of the device 1 is irradiated by the microwave irradiation generated by the microwave source.

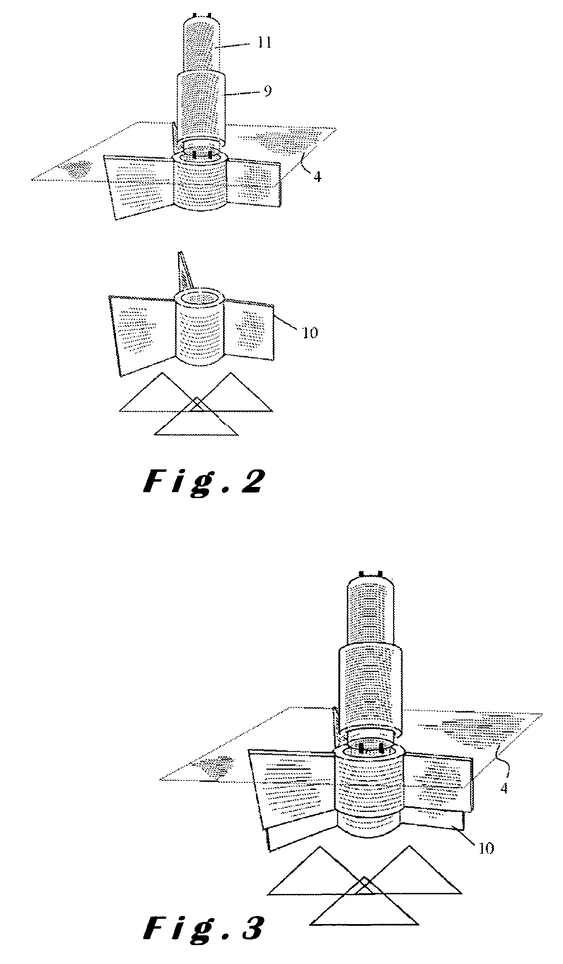

The method according to the invention will be explained hereafter with references to the more schematic figures 2 up to and including 9. Food products or other products or objects are, in case the food products placed in the recipient 3 after preparation in a traditional kitchen or by a mass caterer, preferably in the thereto intended compartments 13 of the recipient 3. The packaging 2 is then preferably first airtightly closed with the closing element 4, for example a foil. Hereby it is noted that the canals 12 provided between the connecting chamber 5 and the recipient remain empty.

After the closing of the packaging 2 by the closing element 4, the content of the recipient 3 will be sterilized by means of the device 1. Hereby, the injection means will, as shown on figure 2, as it were, couple with the connecting chamber 5 to inject fluidum into the connecting chamber 5 or remove fluidum from the connecting chamber 5. Preferably, hereto the injection means will move in a downward direction, for example by moving the head 11 with the injection means 6 downwards. The injection means 6 will thereby pierce the closing element 4. Other possibilities to couple the injection means to the connecting chamber are however possible and are explained hereabove. The coupling preferably is such that after coupling a reduced contact between the content of the recipient 3 and the outer atmosphere is obtained, by which also outside contamination is avoided. Hereto, the head 11 is preferably provided in a manner in which it connects to the upper edge of the connecting chamber 5.

Simultaneously with the coupling with the closing element 4 by the injection means, the pushing device 10 protects the packaging 2 against the forces caused during the creation of an over- or underpressure. For example the pushing device 10, as shown on figure 3, moves upwards to protect the packaging 2. On the other hand the pushing device 10 can always be at the desired position such as the pushing device 10 shown in figure 14. The pushing device 10 shown in figure 1 is positioned near the connecting chamber 5.

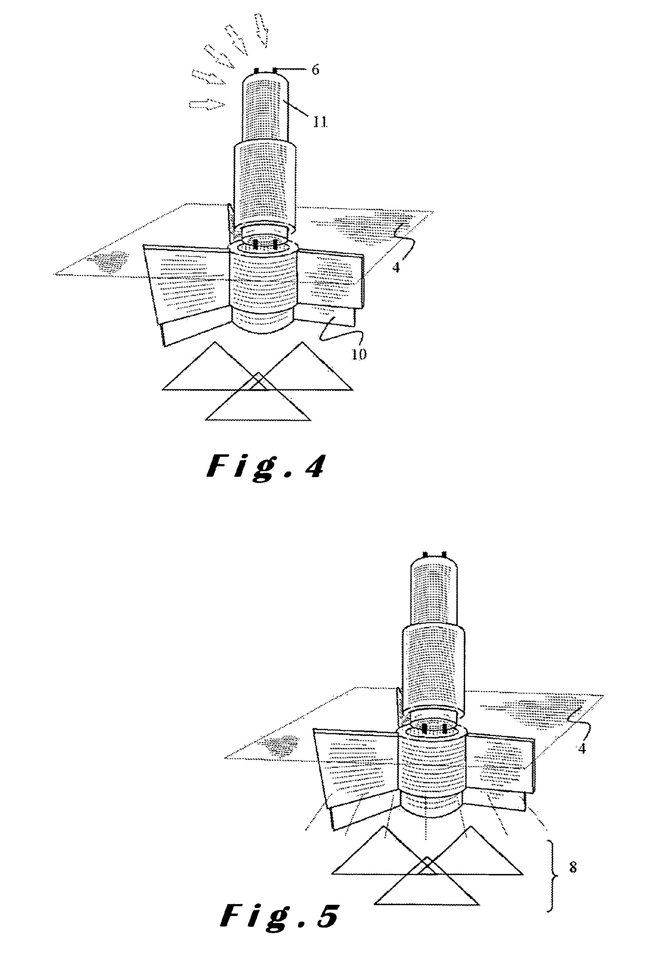

After this the pressure device 7, for example a compressor, will inject the fluidum into the connecting chamber through the injection means 6 (see figure 4) to create an overpressure in the packaging 2. The injected fluidum flows through the canals 12 to the different compartments 13. Once the desired pressure is obtained, the content of the different compartments 13 can be brought to the desired temperature through means of the separate heating parts of the heating element 8 (see figure 5). The duration/ intensity of the heating is controlled separately per compartment 13 by detection means. The results of this detection will determine the duration and the force of the heating in function of the content of the recipient 3. The sterilization process is started by the high pressure, caused by the injected fluidum, and the temperature, caused by the heating element 8, in the different compartments 13 of the recipient 3.

The pressure build up by injecting the fluidum is preferably 2.5 - 3 bar, of which the inventor discovered that good sterilization results can be obtained at a temperature of the content of the recipient 3 of above 100°C, preferably of above 135°C, most preferably 135°C since at such a temperature an improved killing of the micro-organisms is also obtained.

The fluidum injected for sterilization can for example be air, but can also be vapor, steam of even overheated steam. Steam, and with more preference overheated steam can be used to sterilize medical instruments or even empty recipients 3.

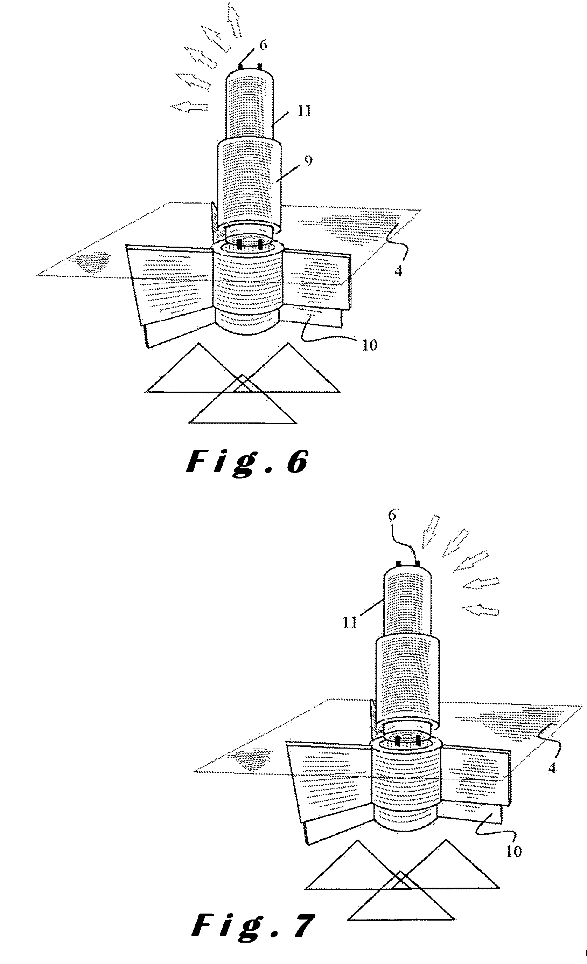

After the complete sterilization process - after all each compartment is preferably sterilized separately - the overpressure and excessive vapor (caused by the sterilization process) are removed by creating an underpressure (see figure 6). To create an underpressure in the recipient 3 the pressure device 7 will remove the air present and possible vapor through the injection means 6 out of the recipient 3 (by suction). After building up of the underpressure in the recipient 3, it is possible to inject a conserving gas in the packaging 2 through the injection means 6 (see figure 7). The conserving gas can be stored in a separate storing unit 15.

It is also possible to cause a transport of fluidum between the first connecting chamber 5, the recipient 3 and the second connecting chamber 27 of the packaging 2, if present. Such a transport of fluidum allows a further improvement of the sterilization and allows rinsing the content of the recipient 3, especially if this contains objects such as medical instruments or if it is empty, preferably with steam, more preferably with overheated steam.

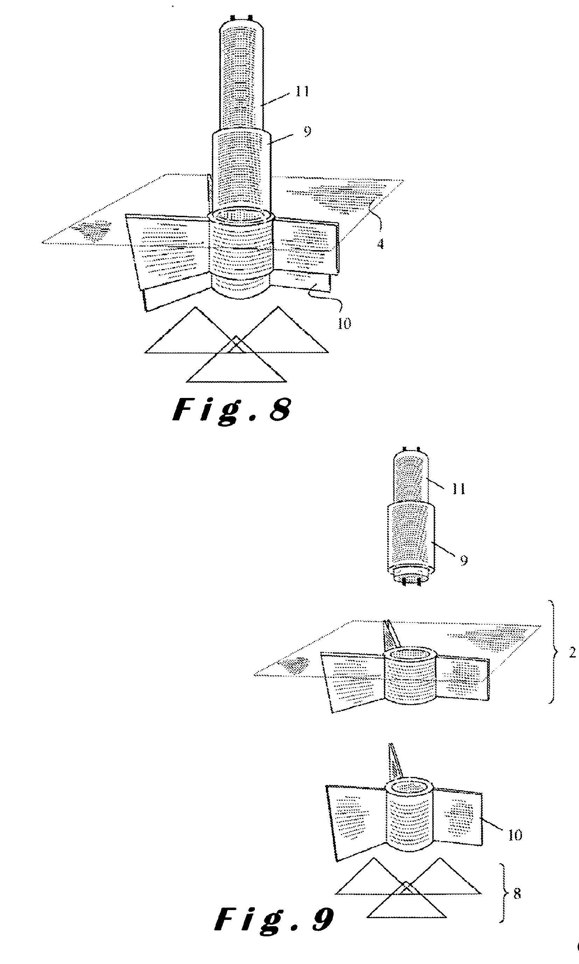

Afterwards a closing unit 9 will slide over the head 11 until the foil 4, and seal the foil near the canals 12 provided in the upper edge of the connecting chamber 5 (see figure 8). The closing unit 9 preferably has a shape corresponding to the shape of the connecting chamber 5, more specifically corresponding to the shape of the upper edge of the connecting chamber 5 so that also the canals 12 provided in the connecting chamber 5 are sealed. After closing the foil, the head with the injection means and the closing unit move upwards, the pushing device moves downward and the packaging is removed out of the device (see figure 9). The packaging can afterwards still be provided with a decorative lid with printing.

## Claims

1. System for sterilization, comprising a packaging (2) comprising a recipient (3) closed with a closing element (4) and an device (1) with a heating element (8) for sterilizing the content of the recipient (3) by heating, **characterized in that** the device (1) comprises a pressure device (7) to create an over- and/or an underpressure in the recipient (3) by injecting a fluidum in and/or removing the fluidum out of the recipient through a connecting chamber (5) present in the packaging (2) and closing means (9) to airtightly close the recipient (3) after sterilization by means of the closing element (4).

2. System for sterilization as claimed in claim 1, **characterized in that** the pressure device (7) comprises injection means (6) to respectively inject the fluidum through the packaging (2) and/or to remove the fluidum from the connecting chamber (5) which is airtightly closable by the closing element (4) connected to the recipient (3) to create the under-and/or overpressure in the recipient (3).

3. System as claimed in claim 1 or 2, **characterized in that** the connecting chamber (5) is a chamber separate from the recipient (3).

4. System as claimed in claim 2 or 3, **characterized in that** the injection means (6) are provided to couple with the connecting chamber (5).

5. System as claimed in any one of claims 2-4, **characterized in that** the injection means (6) are provided on a movable head (11).

6. System as claimed in claim 5, **characterized in that** the closing means comprise a closing unit (9) slidable over the movable head (11), to airtightly close the recipient (3).

7. System as claimed in any one of the preceding claims, **characterized in that** the above mentioned device (1) further comprises a pushing device (10) for exerting a force on the sides of the packaging (2) opposite to the force exerted during the creation of an under- and/or overpressure.

8. System as claimed in claim 7, **characterized in that** the pushing device (10) comprises pushing elements to exert the force on the sides of the packaging (2).

9. System as claimed in any one of preceding claims, **characterized in that** the recipient (3) and the connecting chamber (5) are connected tp each other through a canal (12).

10. System as claimed in claim 9, **characterized in that** the closing unit (9) is provided to close the canal (12) by sealing the closing element (4) with the recipient (3).

11. System as claimed in one of the preceding claims, **characterized in that** the recipient (3) comprises food.

12. Method for sterilization of the content of a recipient (3) of a packaging (2), **characterized in that** the method comprises the following steps:
a. closing the recipient (3) with a closing element (4);
b. creating an over- and/or underpressure in the recipient (3) by injecting the fluidum in and/or removing the fluidum out of the recipient (3) through a connecting chamber (5) present in the packaging (2);
c. heating the content of the recipient (3) to a predetermined temperature to sterilize the content of the recipient:
d. airtight closing of the recipient (3) by means of the closing element (4).

13. Method as claimed in claim 12, **characterized in that** during the heating of the content of the recipient (3) to sterilize the content of the recipient, an overpressure is created in the recipient (3) by injection of the fluidum in the recipient (3).

14. Method as claimed in claim 12 or 13, **characterized in that** for the airtight closing of the recipient (3) an underpressure is created in the recipient through the connecting chamber (5).

15. Method as claimed in any one of claims 12-14, **characterized in that** a transport of the fluidum is caused between the connecting chamber (5), the recipient (3) and a further connecting chamber (27) that is connected airtight closable by the closing element (4) with the recipient (3), by injecting fluidum in the first connecting chamber (5) and removing it from the second connecting chamber (27).

16. Method as claimed in any one of claims 12-15, **characterized in that** before the recipient is closed airtight by means of the closing element (4), a conserving gas is injected in the packaging (2) through injection means (6).

17. Method as claimed in any one of claims 12-16, **characterized in that** for creating the under- and/or overpressure in the recipient (3) by injecting the fluidum in or removing it out of the recipient (3), through the connecting chamber (5), the recipient (3) is already closed airtight by the closing element (4) and **in that** the recipient (3) after heating the content of the recipient (3) on the predetermined temperature to sterilize the content of the recipient is again closed airtight by means of the closing element (4).

18. Device (1) for the sterilization of the content of a recipient (3) of a packaging (2), **characterized in that** this is provided to be applied in a system as claimed in any of claims 1 to 11 and comprises a heating element (8) for sterilizing the content of the recipient (3) by heating, a pressure device (7) to create an over- and/or an underpressure in the recipient (3) by injecting a fluidum in and/or removing the fluidum out of the recipient through a connecting chamber (5) present in the packaging (2) and closing means (9) to airtightly close the recipient (3) after sterilization by means of the closing element (4).

19. Packaging (2) with a connecting chamber (5) provided to create an under- and/or overpressure in a recipient (3) closed by a closing element (4), a first connecting chamber (5) connected to the recipient (3) by a canal (12) and provided to create an under- and/or overpressure in the recipient (3) by injecting a fluidum in and/or removing the fluidum out of the recipient and a closing element (4) for the airtight closing of the recipient (3) after an under- and/or overpressure is created in the recipient (3), **characterized in that** the packaging (2) is provided with a second connecting chamber (27) separate from the first connecting chamber (5) which is airtightly closable connected by the closing element (4) with the recipient (3) in order to cause a transport of the fluidum between the first connecting chamber (5), the recipient (3) and the second connecting chamber (27).

20. Packaging (2) as claimed in claim 19, **characterized in that** the recipient (3) is airtightly closed.

## Patentansprüche

1. System zur Sterilisierung, das ein Packmittel (2) aufweist, welches einen Aufnahmebehälter (3), der mit einem Verschlusselement (4) verschlossen wird, und eine Vorrichtung (1) mit einem Heizelement (8) zum Sterilisieren von dem Inhalt des Aufnahmebehälters (3) durch Erhitzen enthält, **dadurch gekennzeichnet, dass** die Vorrichtung (1) eine Druckvorrichtung (7) zur Erzeugung eines Über- und/oder eines Unterdrucks in dem Aufnahmebehälter (3) durch Einspritzen eines Fluids in und/oder Entfernen des Fluids aus dem Aufnahmebehälter durch eine Verbindungskammer (5), die in dem Packmittel (2) enthalten ist, und Verschlussmittel (9) aufweist, um den Aufnahmebehälter (3) nach der Sterilisierung mittels des Verschlusselements (4) luftdicht zu verschließen.

2. System zur Sterilisierung gemäß Anspruch 1, **dadurch gekennzeichnet, dass** die Druckvorrichtung (7) Einspritzmittel (6) aufweist, um entsprechend das Fluid durch das Packmittel (2) einzuspritzen und/oder das Fluid aus der Verbindungskammer (5) zu entfernen, die durch das Verschlusselement (4) luftdicht verschließbar mit dem Aufnahmebehälter (3) verbunden ist, um den Unter- und/oder Überdruck in dem Aufnahmebehälter (3) zu erzeugen.

3. System gemäß Anspruch 1 oder 2, **dadurch gekennzeichnet, dass** die Verbindungskammer (5) eine Kammer ist, die von dem Aufnahmebehälter (3) getrennt ist.

4. System gemäß Anspruch 2 oder 3, **dadurch gekennzeichnet, dass** die Einspritzmittel (6) vorgesehen sind, an die Verbindungskammer (5) anzukoppeln.

5. System gemäß einem der Ansprüche 2 - 4, **dadurch gekennzeichnet, dass** die Einspritzmittel (6) auf einem beweglichen Kopf (11) vorgesehen sind.

6. System gemäß Anspruch 5, **dadurch gekennzeichnet, dass** die Verschlussmittel eine Verschlusseinheit (9) aufweisen, die über den beweglichen Kopf (11) verschiebbar ist, um den Aufnahmebehälter (3) luftdicht zu verschließen.

7. System gemäß einem der vorhergehenden Ansprüche, **dadurch gekennzeichnet, dass** die oben erwähnte Vorrichtung (1) ferner eine Schiebevorrichtung (10) zum Ausüben einer Kraft auf die Seiten des Packmittels (2) gegen die Kraft, die während der Erzeugung eines Unter-und/oder Überdrucks ausgeübt wird.

8. System gemäß Anspruch 7, **dadurch gekennzeichnet, dass** die Schiebevorrichtung (10) Schiebelemente enthält, um die Kraft auf die Seiten des Packmittels (2) auszuüben.

9. System gemäß einem der vorhergehenden Ansprüche, **dadurch gekennzeichnet, dass** der Aufnahmebehälter (3) und die Verbindungskammer (5) durch einen Kanal (12) miteinander verbunden sind.

10. System gemäß Anspruch 9, **dadurch gekennzeichnet, dass** die Verschlusseinheit (9) vorgesehen ist, den Kanal (12) durch das Abdichten von dem Verschlusselement (4) mit dem Aufnahmebehälter (3) zu schließen.

11. System gemäß einem der vorhergehenden Ansprüche, **dadurch gekennzeichnet, dass** der Aufnahmebehälter (3) Nahrungsmittel enthält.

12. Verfahren zur Sterilisierung von dem Inhalt eines Aufnahmebehälters (3) eines Packmittels (2), **dadurch gekennzeichnet, dass** das Verfahren die folgenden Schritte umfasst:
a. Verschließen des Aufnahmebehälters (3) mit einem Verschlusselement (4);
b. Erzeugen eines Über- und/oder Unterdrucks in dem Aufnahmebehälter (3) durch Einspritzen von dem Fluid in und/oder Entfernen von dem Fluid aus dem Aufnahmebehälter (3) durch eine Verbindungskammer (5), die in dem Packmittel (2) enthalten ist;
c. Erhitzen des Inhaltes von dem Aufnahmebehälter (3) auf eine vorbestimmte Temperatur zur Sterilisierung des Inhaltes von dem Aufnahmebehälter;
d. luftdichtes Verschließen des Aufnahmebehälters (3) mittels des Verschlusselements (4).

13. Verfahren gemäß Anspruch 12, **dadurch gekennzeichnet, dass** während des Erhitzens von dem Inhalt des Aufnahmebehälters (3) zum Sterilisieren des Inhaltes des Aufnahmebehälters, ein Überdruck in dem Aufnahmebehälter (3) durch Einspritzen von dem Fluid in den Aufnahmebehälter (3) erzeugt wird.

14. Verfahren gemäß Anspruch 12 oder 13, **dadurch gekennzeichnet, dass** für das luftdichte Verschließen des Aufnahmebehälters (3) durch die Verbindungskammer (5) in dem Aufnahmebehälter ein Unterdruck erzeugt wird.

15. Verfahren gemäß einem der Ansprüche 12 - 14, **dadurch gekennzeichnet, dass** ein Transport von dem Fluid zwischen der Verbindungskammer (5), dem Aufnahmebehälter (3) und einer weiteren Verbindungskammer (27), die luftdicht verschließbar durch das Verschlusselement (4) mit dem Aufnahmebehälter (3) verbunden ist, durch Einspritzen von Fluid in die erste Verbindungskammer (5) und Entfernen von Fluid aus der zweiten Verbindungskammer (27) hervorgerufen wird.

16. Verfahren gemäß einem der Ansprüche 12 - 15, **dadurch gekennzeichnet, dass** bevor der Aufnahmebehälter luftdicht mittels des Verschlusselements (4) verschlossen wird, durch die Einspritzmittel (6) ein Konservierungsgas in das Packmittel (2) eingespritzt wird.

17. Verfahren gemäß einem der Ansprüche 12 - 16, **dadurch gekennzeichnet, dass** zur Erzeugung eines Unter- und/oder Überdrucks in dem Aufnahmebehälter (3) durch Einspritzen des Fluids in oder Entfernen des Fluids aus dem Aufnahmebehälter (3) durch die Verbindungskammer (5) der Aufnahmebehälter (3) bereits luftdicht durch das Verschlusselement (4) verschlossen ist und dadurch, dass der Aufnahmebehälter (3) nach dem Erhitzen des Inhaltes von dem Aufnahmebehälter (3) auf die vorbestimmte Temperatur zum Sterilisieren des Inhaltes des Aufnahmebehälters wiederum mittels des Verschlusselements (4) luftdicht verschlossen wird.

18. Vorrichtung (1) für die Sterilisierung von dem Inhalt eines Aufnahmebehälters (3) eines Packmittels (2), **dadurch gekennzeichnet, dass** sie vorgesehen ist, in einem System gemäß einem der Ansprüche 1 bis 11 eingesetzt zu werden und ein Heizelement (8) zum Sterilisieren des Inhaltes von dem Aufnahmebehälter (3) durch Erhitzen, eine Druckvorrichtung (7) zur Erzeugung eines Über- und/oder eines Unterdrucks in dem Aufnahmebehälter (3) durch Einspritzen eines Fluids in und/oder Entfernen des Fluids aus dem Aufnahmebehälter durch eine Verbindungskammer (5), welche in dem Packmittel (2) enthalten ist, und Verschlussmittel (9) aufweist, um nach der Sterilisierung den Aufnahmebehälter (3) mittels des Verschlusselements (4) luftdicht zu verschließen.

19. Packmittel (2) mit einer Verbindungskammer (5), die zur Erzeugung eines Unter- und/oder Überdrucks in einem Aufnahmebehälter (3), welcher durch ein Verschlusselement (4) verschlossen ist, vorgesehen ist, einer ersten Verbindungskammer (5), die mit dem Aufnahmebehälter (3) durch einen Kanal (12) verbunden und zur Erzeugung eines Unter-und/oder Überdrucks in dem Aufnahmebehälter (3) durch Einspritzen eines Fluids in und/oder Entfernen des Fluids aus dem Aufnahmebehälter vorgesehen ist, und einem Verschlusselement (4) für das luftdichte Verschließen von dem Aufnahmebehälter (3) nachdem ein Unter- und/oder Überdruck in dem Aufnahmebehälter (3) erzeugt worden ist, **dadurch gekennzeichnet, dass** das Packmittel (2) mit einer zweiten Verbindungskammer (27) versehen ist, getrennt von der ersten Verbindungskammer (5), welche durch das Verschlusselement (4) luftdicht verschließbar mit dem Aufnahmebehälter (3) verbunden ist, um einen Transport von dem Fluid zwischen der ersten Verbindungskammer (5), dem Aufnahmebehälter (3) und der zweiten Verbindungskammer (27) hervorzurufen.

20. Packmittel (2) gemäß Anspruch 19, **dadurch gekennzeichnet, dass** der Aufnahmebehälter (3) luftdicht verschlossen ist.

## Revendications

1. Système de stérilisation, comprenant un conditionnement (2) comprenant un récipient (3) fermé avec un élément de fermeture (4) et un dispositif (1) avec un élément chauffant (8) pour stériliser le contenu du récipient (3) par chauffage, **caractérisé en ce que** le dispositif (1) comprend un dispositif de pression (7) pour créer une surpression et / ou une sous-pression dans le récipient (3) en injectant un fluide dans le récipient et / ou en éliminant le fluide du récipient par l'intermédiaire d'une chambre de connexion (5) présente dans le conditionnement (2) et des moyens de fermeture (9) pour fermer hermétiquement le récipient (3) après stérilisation au moyen de l'élément de fermeture (4).

2. Système de stérilisation tel que revendiqué dans la revendication 1, **caractérisé en ce que** le dispositif de pression (7) comprend des moyens d'injection (6) pour injecter respectivement le fluide à travers le conditionnement (2) et / ou pour éliminer le fluide de la chambre de connexion (5) qui peut être fermée hermétiquement par l'élément de fermeture (4) relié au récipient (3) pour créer la sous-pression et / ou surpression dans le récipient (3).

3. Système tel que revendiqué dans la revendication 1 ou 2, **caractérisé en ce que** la chambre de connexion (5) est une chambre séparée du récipient (3).

4. Système tel que revendiqué dans la revendication 2 ou 3, **caractérisé en ce que** les moyens d'injection (6) sont prévus pour s'accoupler avec la chambre de connexion (6).

5. Système tel que revendiqué dans l'une quelconque des revendications 2 - 4, **caractérisé en ce que** les moyens d'injection (6) sont prévus sur une tête mobile (11).

6. Système tel que revendiqué dans la revendication 5, **caractérisé en ce que** les moyens de fermeture comprennent une unité de fermeture (9) pouvant coulisser sur la tête mobile (11), pour fermer hermétiquement le récipient (3).

7. Système tel que revendiqué dans l'une quelconque des revendications précédentes, **caractérisé en ce que** le dispositif (1) mentionné ci-dessus comprend en outre un dispositif poussoir (10) pour exercer une force sur les côtés du conditionnement (2) opposée à la force exercée pendant la création d'une sous-pression et / ou surpression.

8. Système tel que revendiqué dans la revendication 7, **caractérisé en ce que** le dispositif poussoir (10) comprend des éléments poussoirs pour exercer la force sur les côtés du conditionnement (2).

9. Système tel que revendiqué dans l'une quelconque des revendications précédentes, **caractérisé en ce que** le récipient (3) et la chambre de connexion (5) sont connectés l'un à l'autre par l'intermédiaire d'un canal (12).

10. Système tel que revendiqué dans la revendication 9, **caractérisé en ce que** l'unité de fermeture (9) est prévue pour fermer le canal (12) en connectant hermétiquement l'élément de fermeture (4) au récipient (3).

11. Système tel que revendiqué dans l'une quelconque des revendications précédentes, **caractérisé en ce que** le récipient (3) contient de la nourriture.

12. Procédé pour stériliser le contenu d'un récipient (3) d'un conditionnement (2), **caractérisé en ce que** le procédé comprend les étapes suivantes :
a. fermer le récipient (3) avec un élément de fermeture (4) ;
b. créer une surpression et / ou sous-pression dans le récipient (3) en injectant le fluide dans le récipient (3) et / ou en enlevant le fluide du récipient par l'intermédiaire d'une chambre de connexion (5) présente dans le conditionnement (2) ;
c. chauffer le contenu du récipient (3) à une température prédéterminée pour stériliser le contenu du récipient ;
c. fermer hermétiquement le récipient (3) au moyen de l'élément de fermeture (4).

13. Procédé tel que revendiqué dans la revendication 12, **caractérisé en ce que** pendant le chauffage du contenu du récipient (3) pour stériliser le contenu du récipient, une surpression est créée dans le récipient (3) par injection du fluide dans le récipient (3).

14. Procédé tel que revendiqué dans la revendication 12 ou 13, **caractérisé en ce que** pour la fermeture hermétique du récipient (3), une sous-pression est créée dans le récipient par l'intermédiaire de la chambre de connexion (5).

15. Procédé tel que revendiqué dans l'une quelconque des revendications 12 - 14, **caractérisé en ce qu'**un transport du fluide est provoqué entre la chambre de connexion (5), le récipient (3) et une autre chambre de connexion (27) qui est reliée de manière hermétiquement fermable par l'élément de fermeture (4) au récipient (3), en injectant du fluide dans la première chambre de connexion (5) et en l'enlevant de la deuxième chambre de connexion (27).

16. Procédé tel que revendiqué dans l'une quelconque des revendications 12 - 15, **caractérisé en ce qu'**avant que le récipient soit hermétiquement fermé au moyen de l'élément de fermeture (4), un gaz de conservation est injecté dans le conditionnement (2) par l'intermédiaire de moyens d'injection (6).

17. Procédé tel que revendiqué dans l'une quelconque des revendications 12 - 16, **caractérisé en ce que** pour créer la sous-pression et / ou surpression dans le récipient (3) en injectant le fluide dans le récipient (3) et en l'éliminant du récipient, par l'intermédiaire de la chambre de connexion (5), le récipient (3) est déjà fermé hermétiquement par l'élément de fermeture (4) et **en ce que** le récipient (3), après avoir chauffé le contenu du récipient (3) à la température prédéterminée pour stériliser le contenu du récipient, est de nouveau fermé hermétiquement au moyen de l'élément de fermeture (4).

18. Dispositif pour la stérilisation du contenu d'un récipient (3) d'un conditionnement (2), **caractérisé en ce qu'**il est prévu pour être appliqué dans un système tel que revendiqué dans l'une quelconque des revendications 1 à 11 et comprend un élément chauffant (8) pour stériliser le contenu du récipient (3) par chauffage, un dispositif de pression (7) pour créer une surpression et / ou sous-pression dans le récipient (3) en injectant un fluide dans le récipient et / ou en éliminant le fluide du récipient par l'intermédiaire d'une chambre de connexion (5) présente dans le conditionnement (2) et des moyens de fermeture (9) pour fermer hermétiquement le récipient (3) après stérilisation au moyen de l'élément de fermeture (4).

19. Conditionnement (2) avec une chambre de connexion (5) prévue pour créer une sous-pression et / ou surpression dans un récipient (3) fermé par un élément de fermeture (4), une première chambre de connexion (5) reliée au récipient (3) par un canal (12) et prévue pour créer une sous-pression et / ou surpression dans le récipient (3) en injectant un fluide dans le récipient et / ou en éliminant le fluide du récipient et un élément de fermeture (4) pour la fermeture hermétique du récipient (3) après qu'une sous-pression et / ou surpression a été créée dans le récipient (3), **caractérisé en ce que** le conditionnement (2) est pourvu d'une deuxième chambre de connexion (27) séparée de la première chambre de connexion (5) qui peut être hermétiquement fermée et est reliée par l'élément de fermeture (4) au récipient (3) afin de provoquer un transport du fluide entre la première chambre de connexion (5), le récipient (3) et la deuxième chambre de connexion (27).

20. Conditionnement (2) tel que revendiqué dans la revendication 19, **caractérisé en ce que** le récipient (3) est hermétiquement fermé.
